# EUROPEAN PATENT SPECIFICATION

(11) **EP 3 286 286 B1**
(45) Date of publication and mention of the grant of the patent: **06.03.2019**
(21) Application number: 16724490.4
(22) Date of filing: 12.04.2016
(51) Int. Cl.: C10G 33/08, C10G 31/08, G01N 23/204, G01F 23/288, G01T 3/00, C10G 32/04, G01N 33/28

(54) **DESALTING METHOD AND APPARATUS**
ENTSALZUNGSVERFAHREN UND -VORRICHTUNG
PROCÉDÉ ET APPAREIL DE DESSALAGE

(30) Priority: 21.04.2015 US 201562150333 P
(43) Date of publication of application: 28.02.2018
(73) Proprietor: BP Corporation North America Inc., Houston, TX 77079 (US)
(72) Inventor: HACKETT, Craig, Naperville, IL 60563 (US); ENGLISH, Jason, Naperville, IL 60563 (US)
(74) Representative: Hamer, Christopher K.
(86) International application number: PCT/US2016/027082
(87) International publication number: WO 2016/171952

(56) References cited:
- US-A- 5 219 471
- US-A1- 2014 202 929
- Sudan Academy ET AL: "Investigation of Process Equipment in Petrochemical Industry", , 30 April 2007 (2007-04-30), XP055282241, Retrieved from the Internet: URL:http://www.iaea.org/inis/collection/NC LCollectionStore/_Public/38/096/38096986.p df [retrieved on 2016-06-21]

## Description

### FIELD OF THE INVENTION

The invention relates to the desalting of a hydrocarbon feedstock, such as crude oil. In particular, the invention relates to a method and apparatus for optimizing a desalting process.

### BACKGROUND OF THE INVENTION

When crude oil is extracted from a reservoir, it contains water and salts. At the high temperatures that may be encountered in a refinery during crude oil processing, the water can hydrolyze the salts to form corrosive acids. Chloride salts are typically found in crude oil and pose a particular problem, since they can form hydrochloric acid. Bromide salts can also be found, and they can form hydrobromic acid.

Over time, corrosive acids can cause significant damage to refinery equipment. Damage is commonly observed in the lines that transport crude oil from one area of a refinery to another. Considerable time and cost may be involved in replacing damaged refinery equipment. In some cases, for instance where a bypass pipe has not been provided, processing of the crude oil will need to be stopped entirely in order for the refinery equipment to be replaced.

It is therefore desirable for salts to be removed from hydrocarbon fluids such as crude oil before refinery processing. To solve this problem, crude oils are typically passed to a desalter before they are processed in a refinery.

Crude oils are typically mixed with wash water before they are passed to a desalter. Once introduced into the desalter, a desalted crude oil phase and an aqueous phase form. The aqueous phase contains water (that which was present in the extracted crude oil, as well as water that has been added to the hydrocarbon stream during processing, such as wash water) and salt. A rag layer separates the two phases. The rag layer is a mixture of the aqueous phase and the desalted crude oil phase.

A desalted crude oil stream and an aqueous stream are withdrawn from the desalter through separate lines. The streams are typically withdrawn at points in the desalter which are a distance from the rag layer so as to minimize the presence of any aqueous components in the desalted crude oil stream and vice versa.

Methods are known for optimizing desalting processes. For instance, demulsifiers are often added to minimize the rag layer and encourage the formation of separate hydrocarbon and aqueous phases. The application of an electrostatic field to the desalting unit may also be used to encourage the formation of separate phases.

It is known that desalting may be optimized by increasing the level of contact between a hydrocarbon stream and wash water to enhance the efficacy of a desalting process. Techniques for improving the mixing between a hydrocarbon stream and wash water include passing the hydrocarbon stream and wash water through a mixing valve. US 2014/0202929 discloses a method for optimizing a desalting process.

Current methods for evaluating mixing between crude oil and wash water in the feed to a desalter rely on theoretical calculations. However, such methods are limited by the accuracy of the input data.

Accordingly, there remains a need for further improvements in desalting processes.

### SUMMARY OF THE INVENTION

The present invention provides a method for optimizing a desalting process in which a hydrocarbon feedstock is passed to a desalter through a line under a set of conditions, the hydrocarbon feedstock containing a hydrocarbon fluid, water and a salt, said method comprising:
obtaining spectra of the hydrocarbon feedstock under a plurality of environments in the line;
comparing the spectra; and
based on the comparison of the spectra, either modifying or maintaining the set of conditions under which the hydrocarbon feedstock is passed to the desalter;
wherein the spectra are obtained using neutron backscattering.

In a further aspect, there is provided an apparatus comprising:
a desalter;
a line through which a hydrocarbon feedstock is passed to the desalter, the hydrocarbon feedstock containing a hydrocarbon fluid, water and a salt; and
a neutron backscatter spectrometer positioned so as to obtain a spectrum of the hydrocarbon feedstock in the line.

In a further aspect, there is provided the use of neutron backscattering as defined in claim 15.

### BRIEF DESCRIPTION OF THE DRAWINGS

**Fig.1** depicts a line feeding a desalter, the line comprising two wash water inlets and two mixing valves;
**Figs. 2a****-e** depict spectra obtained from along the line shown in Fig. 1;
**Figs. 3a****-b** depict spectra obtained from around the wash water valves shown in Fig. 1;
**Fig. 4** depicts a line feeding a desalter, the line comprising a single wash water inlet and two mixing valves; and
**Figs. 5a****-c** depict spectra obtained from the line shown in Fig. 4.

### DETAILED DESCRIPTION OF THE INVENTION

It has now been recognized that that neutron backscattering may be used to determine the extent to which a hydrocarbon stream and water are mixed before they are introduced into a desalter.

Neutron backscattering is a technique in which high energy neutrons are emitted from a neutron backscatter spectrometer and directed into a material. When the high energy neutrons collide with hydrogen nuclei in a material, their energy is reduced. The degree to which the energy of the neutrons is reduced depends on the nature of the material with which they collide. A detector in the neutron backscatter spectrometer detects the reduced energy neutrons. Accordingly, neutron backscattering may be used to measure the 'hydrogen richness' of a material.

Since water and hydrocarbon fluids have different hydrogen richness, by comparing spectra obtained using neutron backscattering, an indication of the degree of mixing between the hydrocarbon fluid and water in different environments may be obtained. Accordingly, neutron backscattering allows the desalting process to be analyzed and, if necessary, optimized. Moreover, by using neutron backscattering, the degree of mixing between the hydrocarbon fluid and water may be evaluated during operation of a desalting process, and without having to take a sample of hydrocarbon feedstock from the line.

The hydrocarbon feedstock may be any refinery feedstock. The hydrocarbon feedstock may be selected from a crude oil, a synthetic crude, a biocomponent, an intermediate stream such as a residue, gas oil, vacuum gas oil, naphtha and cracked stocked, and blends thereof. For instance, a blend of one or more crude oils or a blend of one or more crude oils with a synthetic crude may be used. Typically, the hydrocarbon feedstock will comprise a crude oil.

The water that is present in the hydrocarbon feedstock may be residual water that is present in the hydrocarbon feedstock. For instance, where the hydrocarbon feedstock comprises crude oil, brine may be present in the crude oil from extraction from a reservoir. Alternatively, residual water may be present in the hydrocarbon feedstock, for instance from a previous desalting process.

Water will typically be present in the hydrocarbon feedstock in an amount of less than 10 % by weight, less than 5%, such as around 3% by weight of the hydrocarbon feedstock. It will be appreciated that these amounts do not include further wash water that is typically added to the hydrocarbon feedstock along the line to the desalter.

The hydrocarbon feedstock also comprises a salt. The salt may be an inorganic salt. The salt may be selected from alkali and alkaline earth metal salts, such as alkali and alkaline earth metal halides. Typical salts which may be found in hydrocarbon feedstocks include sodium chloride, potassium chloride and magnesium chloride. Crude oil typically contains sodium chloride. Potassium chloride and magnesium chloride may also be found in crude oil, though typically in smaller amounts than sodium chloride. In instances of the invention, the hydrocarbon feedstock comprises sodium chloride.

The amount of salt that is present will vary between different hydrocarbon feedstocks. The hydrocarbon feedstock will typically contain one or more inorganic chlorides in a total amount of 1-300 ppm, such as 2-100 ppm.

Further components that are typically found in a refinery feedstock may also be present in the hydrocarbon feedstock. For instance, where the hydrocarbon feedstock comprises crude oil, asphaltenes will typically be present.

According to the invention, spectra are obtained using neutron backscattering. A neutron backscatter spectrometer has a neutron generator and a neutron detector. During use, neutrons are directed through the hydrocarbon feedstock. As the neutrons pass through hydrogenous material, the energy of the neutrons decreases. The degree to which the energy decreases depends on the hydrogenous material encountered by the neutrons. Since water and hydrocarbon fluids modify the scatter of neutrons differently, neutron backscattering may be used to assess the degree of mixing between water and a hydrocarbon fluid.

The spectra may be obtained by positioning the neutron backscatter spectrometer against the line through which the hydrocarbon fluid passes. The line through which the hydrocarbon fluid passes will typically have a substantially circular cross-section. In some instances, the neutrons emitted from the neutron backscatter spectrometer will penetrate the line through which the hydrocarbon fluid passes to a depth of from 30-100 % of the diameter of the line. In this way, mixing may be assessed across a significant proportion, if not all, of the cross-section of the line.

According to the invention, spectra are obtained under a plurality of environments. An environment is defined by location and time. Accordingly, environments differ by virtue of location and/or by virtue of the time at a particular location. It will be understood that a reference to a plurality of environments means two or more environments which differ by virtue of their location and/or the time at a particular location. A reference to *e.g.* four environments means that the four environments each different from one another by virtue of their location and/or the time at a particular location
Spectra may be taken at two environments in the line, or more than two environments in the line. Spectra may be obtained at four or more environments in the line such as at eight or more environments in the line. A larger number of spectra gives a more complete picture of the mixing between the hydrocarbon fluid and water.

Spectra may be obtained at a plurality of locations on the line. In this instance, a plurality of neutron backscatter spectrometers may be positioned on the line in the apparatus. A single neutron backscatter spectrometer may alternatively be used to obtain spectra at a plurality of locations on the line.

In some instances, spectra are obtained at longitudinally spaced locations along the line. It will be understood that longitudinally spaced locations are locations spaced from one another along the length of the line, in the direction of flow of the hydrocarbon fluid.

The longitudinally spaced locations may be in substantially the same radial position on the line. For instance, longitudinally spaced locations may be positioned substantially at the top of the line, or longitudinally spaced locations may be positioned substantially at the bottom of the line.

A plurality of neutron backscatter spectrometers may be positioned on the line at longitudinally spaced locations. Alternatively, a single neutron backscatter spectrometer may be used to obtain spectra at each of the longitudinally spaced locations. In this case, the spectrometer must be moved for each spectrum. This means that the longitudinally spaced spectra will be obtained over a period of time.

Spectra obtained at longitudinally spaced locations may give an indication of how the degree of mixing between the hydrocarbon fluid and water varies along the line. This is particularly useful where there is a change in the flow along the line, for instance due to a mixing valve or a wash water inlet.

The line feeding the desalter will typically comprise a wash water inlet.

Wash water may be introduced into the line through the wash water inlet in an amount of 1-30%, preferably 3-20%, and more preferably 5-10% by weight of hydrocarbon stream.

In some instances, a plurality of wash water inlets may introduce the wash water into the line. In these instances, the amounts referred to above relate to the total amount of wash water that is introduced into the line.

In some instances, spectra are obtained at a location upstream of a wash water inlet and at a location downstream of a wash water inlet. Where the wash water and hydrocarbon fluid have been mixed so that there are no distinct phases, minimal differences should be observed between the spectra. Accordingly, smaller spectral differences indicate better mixing, whereas larger spectral differences indicate inferior mixing.

In some instances, the line may comprise a mixing valve.

The flow through the mixing valve may be at a speed of from 0.01-30 m/s, preferably from 0.1-20 m/s, more preferably from 0.5-10 m/s.

In some instances, a plurality of mixing valves may be present along the line. In these instances, the flow speeds referred to above represent the flow speed through each of the mixing valves.

Spectra may be obtained at a location upstream of a mixing valve and at a location downstream of the mixing valve. If the degree of mixing between the wash water and hydrocarbon fluid is the same upstream and downstream of the mixing valve, then minimal differences should be observed between the spectra. This may indicate that the mixing valve is ineffective. Alternatively, it may indicate that the mixing valve is unnecessary as thorough mixing of the hydrocarbon fluid and water is achieved upstream the mixing valve. In order to determine more about the system, it may be desirable to obtain further spectra.

Accordingly, in some instances, the line may comprise a mixing valve and a wash water inlet. The wash water inlet will typically be positioned upstream of the mixing valve.

Spectra may be obtained at a location upstream of the wash water inlet and at a location downstream of the mixing valve. In some instances, spectra may be obtained at longitudinally spaced locations including upstream of a wash water inlet, downstream of the wash water inlet but upstream of a mixing valve, and downstream of the mixing valve. By comparing the spectra, the efficacy of the mixing valve may be assessed. As mentioned above, where the wash water and hydrocarbon fluid have been mixed so that there are no distinct phases, minimal differences should be observed as compared to a spectrum of the hydrocarbon fluid upstream of the wash water inlet.

In some instances, spectra are obtained at radially spaced locations around the line. It will be understood that radially spaced locations are locations positioned in substantially the same cross-sectional plane of the line. The cross-sectional plane lies substantially perpendicular to the flow of the hydrocarbon fluid.

A neutron backscatter spectrometer may be positioned on the line at each of a plurality of radially spaced locations in the apparatus. Alternatively, a single spectrometer may be used to obtain the spectra. In this case, the spectrometer must be moved for each spectrum. This means that the radially spaced spectra will be obtained over a period of time.

Spectra obtained at radially spaced locations may give an indication of how the degree of mixing between the hydrocarbon fluid and water varies at different locations over a cross-section in the line. For instance, spectra could be used to determine whether there is the same proportion of water in the hydrocarbon feedstock in an upper portion of the line and in a lower portion of the line. Differences between spectra obtained at radially spaced locations indicate that there is not uniform mixing across a cross-section of the line.

Typically radially spaced spectra will be obtained at a location downstream of a wash water inlet, since this is where cross-sectional variations in mixing will often occur.

At least four spectra may be obtained at radially spaced locations around the line, such as at least eight spectra. A larger number of radially spaced spectra gives a more complete picture of mixing of the hydrocarbon fluid and water across a cross-section of the line.

The spectra may be obtained at radially spaced locations which are equally spaced around the line. For instance, where two spectra are obtained, they may be obtained on opposite sides of the line from one another, *i.e*. they are separated by an angle of 180°. Where five spectra are obtained, they may be obtained from locations around the line which are separated from one another by an angle of 72°. The spectra may be obtained at locations which are separated from one another by 1-20 cm, such as from 3-10 cm.

In some instances, the line may not be accessible from all sides, in which case spectra may be obtained at radially spaced locations around a portion of the line. The spectra may be obtained at radially spaced locations whereby each of the radially spaced locations fall within an angle of from 45-360°, preferably from 90-360° and more preferably from 180-360°.

In order to get a more complete picture of hydrocarbon fluid and water mixing along the length of the line, a plurality of sets of spectra may be obtained at longitudinally spaced locations on the line, each set of spectra obtained at radially spaced locations on the line. In this way, an indication of how the degree of mixing between the hydrocarbon fluid and water varies along the line and over a cross-section of the line may be obtained.

In some instances, spectra may be obtained at radially spaced locations upstream of a wash water inlet and at radially spaced locations downstream of a wash water inlet. Similarly, spectra may be obtained at radially spaced locations upstream of a mixing valve and at radially spaced locations downstream of a mixing valve.

Spectra obtained at radially spaced locations upstream of a wash water inlet and at radially spaced locations downstream of a mixing valve are particularly useful. In instances, spectra may be obtained at radially spaced locations upstream of a wash water inlet, at radially spaced locations downstream of the wash water inlet but upstream of a mixing valve, and at radially spaced locations downstream of the mixing valve.

Spectra may also be obtained from the same location on the line. In these instances, the spectra are obtained at different points in time. This is particularly useful when there has been a change in the system, and spectra are obtained before and after the change. Changes include adjustments to the amount of wash water that is added to the line, adjustments to the operation of the mixing valve such as changes in pressure drop or blade design, and adjustments to the temperature in the line.

In some instances, spectra will be obtained from the same location on the line before and after addition of wash water to the line. It will be appreciated that, in these instances, it is useful to obtain the spectra downstream of the wash water inlet. Minimal differences between the spectra indicate good mixing of the wash water and the hydrocarbon fluid.

Where spectra are obtained from a plurality of locations on the lines, for instance as described above, then a plurality of spectra may be obtained at each location at different points in time.

Spectra may be obtained to assess the influence of features of the line other than the wash water inlet and mixing valve on mixing of hydrocarbon fluid with water. For instance, emulsion controlling additives may be introduced into the line via an additive inlet. Spectra may be obtained upstream and downstream of the additive inlet. Spectra may also be obtained at a location downstream of the additive inlet, before and after the emulsion controlling additives have been introduced into the line.

The step of comparing the spectra involves identifying differences in the spectra. As discussed above, differences between spectra may indicate that inadequate mixing of the hydrocarbon fluid and water is occurring. If differences are identified, then the set of conditions under which the hydrocarbon feedstock is passed to the desalter may be modified. The conditions are modified in an attempt to improve mixing of the hydrocarbon fluid and water.

It will be appreciated that differences in spectra may be more easily interpreted when there are fewer differences between the environments under which the spectra are taken.

In some instances, substantial differences may not be identified on comparing the spectra. In these instances, the set of conditions under which the hydrocarbon feedstock is passed to the desalter may nonetheless be modified. As will be appreciated, if differences are not identified between spectra then adequate mixing between the hydrocarbon fluid and water may have been achieved already. Accordingly, the conditions are modified in an attempt to improve the efficiency of the desalting process (e.g. by reducing the cost of the process) whilst maintaining adequate mixing of the hydrocarbon fluid with water.

The cost of the desalting process may be reduced by lowering the energy input into the line. This may be achieved by reducing the pressure drop in a mixing valve, or by reducing the temperature in the line through which the hydrocarbon feedstock is passed.

Once the set of conditions under which the hydrocarbon feedstock is passed to the desalter has been modified, the method of the invention may comprise testing the effect of the modified conditions. Testing may comprise obtaining further spectra of the hydrocarbon feedstock. The spectra may be used to determine whether the modified conditions have an effect on mixing of the hydrocarbon fluid with water.

Alternatively, the effect of the modified conditions may be tested by measuring the salt content of the crude oil exiting the desalter. A reduction in salt content indicates that the modified conditions have improved mixing of the hydrocarbon fluid with water. Methods for measuring the salt content of crude oil exiting a desalter are known in the art. An increase in salt content indicates that the modified conditions have reduced mixing of the hydrocarbon fluid with water.

If the modified conditions affect mixing of the hydrocarbon fluid with water, the modified conditions may be maintained, reversed or further modified. Accordingly, it can be seen that the method of the invention may be an iterative process for optimizing the desalting of a hydrocarbon feedstock. In one instance, the steps of obtaining spectra, comparing spectra and modifying conditions are repeated at least 3 times, such as least 5 times. It will be understood that different conditions may be modified in each iteration.

The set of conditions under which the hydrocarbon feedstock is passed to the desalter may be modified by making changes to the wash water inlet or by making changes to the mixing valve. Changes to the wash water inlet include adjusting the amount of wash water that is added and adjusting the wash water injection device (*e.g.* size shape or shape of nozzle, type of device). Another potential change to the water inlet could be modifying its location, e.g. its location relative to the mixing valve. Changes to the mixing valve include adjusting the pressure drop of the mixing valve, adjusting the location of the mixing valve, adjusting the number of mixing valves, adjusting the mixing valve device (*e.g.* size or type of device), adjusting the degree to which the mixing valves are open, addition or removal of a static mixer in addition to the mix valve, etc.

Other changes to the set of conditions may include adjustments to the additive components (*e.g.* emulsion controlling additives) introduced into the line. Such changes may include adjustments to the chemical composition of the additive components, adjustments to the amount of additive components introduced, adjustments to the location at which the additive components are introduced into the line.

Further changes to the set of conditions may include adjustments to the temperature and pressure in the line.

Changes may also be made to the hydrocarbon feedstock, for instance the speed at which the hydrocarbon feedstock is passed through the line or the amount of hydrocarbon feedstock that is passed to the desalter.

The method of the present invention may be used to optimize the desalting of a hydrocarbon feedstock in a desalting process. In some instances, the method of the present invention optimizes desalting by increasing the proportion of salt that is removed from the hydrocarbon fluid during desalting process. An optimized desalting process preferably reduces the total inorganic chloride concentration to less than 5 ppm. Where the desalting process is a two stage process, the total inorganic chloride concentration may be reduced to less than 2 ppm. The desalting process may also be optimized by improvements in efficiency. Improvements in efficiency include increases in throughput, decreases in the energy used to carry out the desalting process and decreases in the cost of the apparatus used to carry out the desalting process.

Any conventional desalter design may be used in the invention. A desalter will typically have a feedstock inlet, a hydrocarbon outlet and an aqueous outlet. In the process of the invention, the hydrocarbon fluid, water and salt are introduced into the desalter via the feedstock inlet. A hydrocarbon phase is removed from the desalter via the hydrocarbon outlet. An aqueous phase is removed from the desalter via the aqueous outlet.

An electric field may be applied to the desalter. This improves the separation of the aqueous phase from the hydrocarbon phase.

The hydrocarbon feedstock may be passed to the desalter in an amount of from 100-100,000 barrels per hour, preferably from 500-50,000 barrels per hour, more preferably from 1,000-20,000 barrels per hour.

Multiple desalting stages may be present in the desalting process. The method of the present invention may involve the steps of obtaining spectra, comparing the spectra and optionally modifying conditions, as described herein, on two or more lines, each line feeding a desalter. The apparatus of the present invention may comprise two or more desalters, each fed by a line, and a neutron backscatter spectrometer.

The invention will now be described with reference to the accompanying figures and examples, in which:

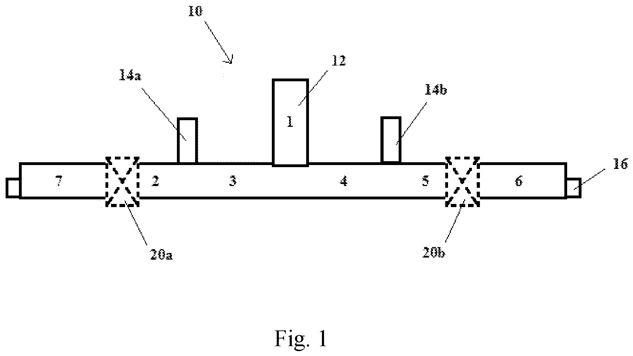

The line (10) shown in Fig. 1 feeds a desalter in a desalting process. The line (10) comprises a hydrocarbon feedstock inlet (12), two wash water inlets (14a, 14b) and two mixing valves (20a, 20b). The crude oil is passed to the desalter via a pipe (16).

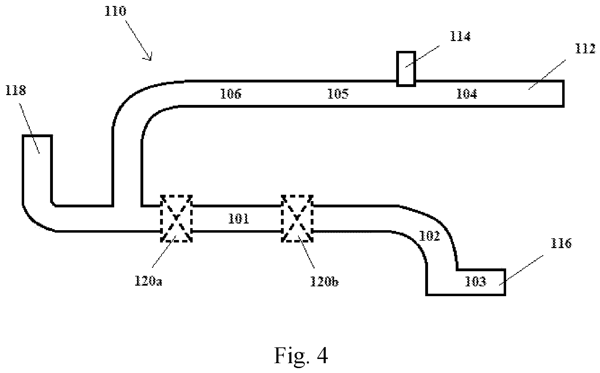

The line (110) shown in Fig. 4 feeds a desalter in a desalting process. The line (110) comprises a hydrocarbon feedstock inlet (112), a single wash water inlet (114) and two mixing valves (120a and 120b). The crude oil is passed to the desalter via a pipe (116). A bypass pipe (118) is also present on the line (110).

### EXAMPLES

In order to understand wash water contact with raw crude oil, spectra were obtained using neutron backscattering on a line feeding a first stage desalter and a line feeding a second stage desalter. The crude oil product stream from the first stage desalter served as the crude oil feedstock for the second stage desalter. The line to the first stage desalter had an arrangement as shown in Fig. 1 and the line to the second stage desalter had an arrangement as shown in Fig. 4.

Spectra were obtained at a number of locations (1-7 in Fig. 1; 101-106 in Fig. 4) along the lines, including upstream of wash water inlets, downstream of wash water inlets, upstream of mixing valves and downstream of mixing valves. Readings were taken at a point in time before wash water was added to the line, and at a point in time after wash water was added to the line.

To obtain the spectra, the neutron backscatter spectrometer was held against the side of the line under inspection. For spectra from radially spaced locations, the neutron backscatter spectrometer moved around the circumference of the line. Readings were taken approximately at 5-7.5 cm intervals around the line. In some cases, counts were only able to be taken 180° from the top of the line due to limited access. The lines were 40 cm in diameter, and the neutron backscatter spectrometer was capable of obtaining data approximately 20 cm into the line.

The results of the neutron backscatter scans at the various locations are shown as counts, representing the neutrons detected. The backscatter counts were compared using radar plots. The radar plots were graphed with the backscatter counts plotted around the line.

Where the crude oil and wash water were intimately mixed, the difference between the number of neutrons detected in raw crude oil (*i.e.* before wash water addition) and the number of neutrons detected in crude oil containing wash water was small. An increase in the count difference indicates that the mixing was poor, and could be improved.

Example 1 - analysis of the line in the first desalting process Operating conditions at the time of sampling in the first stage are shown in Table 1:

| | Wash water rate (barrels / hour) | Amount of wash water added (% by volume of crude oil) | Delta pressure (psi) | Valve open (%) | Crude flow (barrels / hour) | Crude velocity (m/s) |
|---|---|---|---|---|---|---|
| East mixing valve | 255 | 5.5 | 16 | 19.5 | 4,625 | 1.55 |
| West mixing valve | 264 | 5.7 | 15.8 | 16.5 | 4,625 | 1.55 |

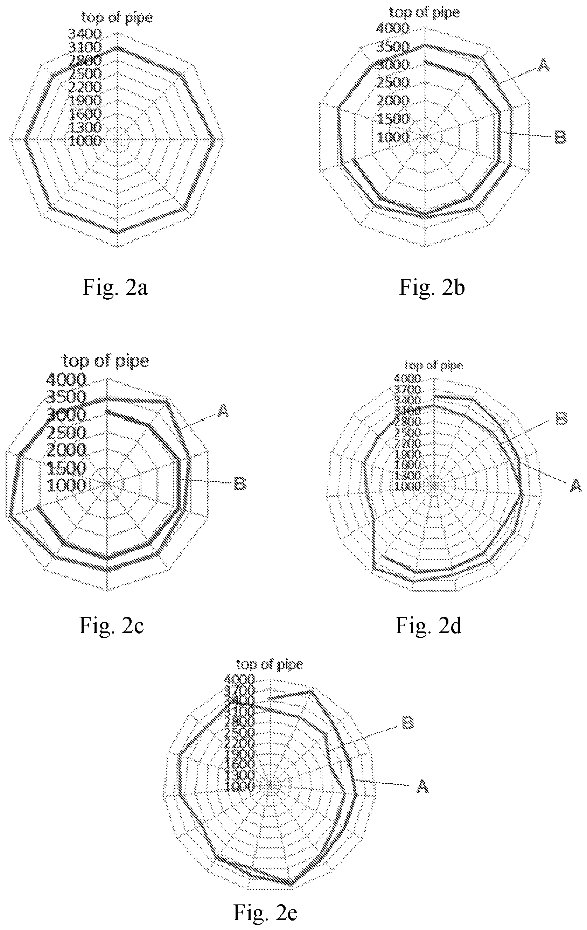

The radar plot shown in Fig. 2a shows the results of spectra obtained at the crude oil inlet (see location 1 of Fig. 1), *i.e.* upstream of the wash water inlet. The crude oil contained 3% residual water. This water was injected upstream of cold preheat exchangers. The radar plot shows that the oil and water were mixed uniformly with no distinct water or crude oil phase.

The radar plots shown in Figs. 2b-e show the results of spectra obtained at a number of locations (locations 5, 2, 6 and 7 as shown in Fig. 1, respectively). Spectra were obtained at a point in time before the addition of wash water to the system (denoted in Figs. 2b-e by 'B'), as well as at a point in time after the addition of wash water to the system (denoted in Figs. 2b-e by 'A').

It can be seen from Figs. 2b-c that the wash water and crude oil were not well mixed at locations 5 and 2, respectively, *i.e.* downstream of a wash water inlet but upstream of a mixing valve.

It can be seen from Figs. 2d-e that the wash water and crude oil were still imperfectly mixed at locations 6 and 7, respectively, *i.e.* downstream of a mixing valve.

The radar plots shown in Figs. 2b-e also show that the highest counts were generally towards the bottom of the line, indicating that the water concentration was highest at the bottom of the line both before and after the mixing valves.

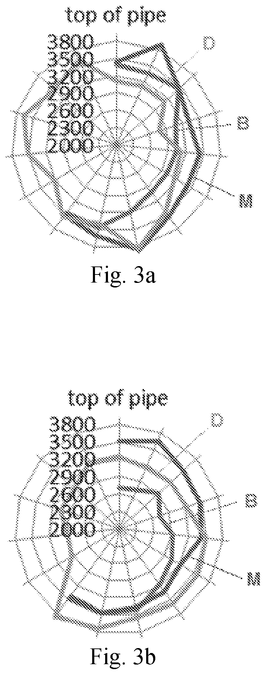

The radar plots shown in Fig. 3a show the results of spectra obtained around the east mixing valve (20b in Fig. 1), and the radar plots shown in Fig. 3b show the results of spectra obtained around the west mixing valve (20a in Fig. 1). The radar plots shown in Figs. 3a-b show the results of spectra obtained at a point in time before the addition of wash water to the system (denoted in Figs. 3a-b by 'B'), at a point in time after the addition of wash water to the system, downstream of the wash water inlet but upstream of the mixing valve (denoted in Figs. 3a-b by 'M'), and at a point in time after the addition of wash water to the system, downstream of the mixing valve (denoted in Figs. 3a-b by 'D').

The radar plots shown in Fig. 3a-b show that there is a distinct water phase on the bottom of the line after wash water injection and after the mixing valve.

These results suggest that mixing could be improved. It was decided that the system could be modified by use of a smaller mixing valve and a wash water injection quill.

Example 2 - analysis of the line in the second desalting process Operating conditions at the time of sampling in the second stage are shown in Table 2:

| | Wash water rate (barrels / hour) | Amount of wash water added (% by volume of crude oil) | Delta pressure (psi) | Valve open (%) | Crude flow (barrels / hour) | Crude velocity (m/s) |
|---|---|---|---|---|---|---|
| Mixing valve | 475 | 5.1 | 16-16.5 | 25.0 | 9,250 | 3.14 |

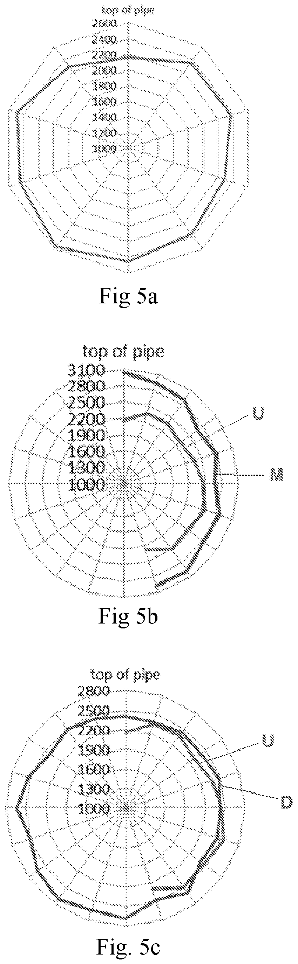

The radar plot shown in Fig. 5a shows the results of spectra obtained at the crude oil inlet (see location 4 of Fig. 4), *i.e.* upstream of the wash water inlet. It can be seen that the counts obtained from the line in the second stage were lower than those obtained from the line in the first stage. This difference was due to the amount of water in the crude feeds. Whilst the first stage raw crude oil included 3% residual water, the second stage crude oil feed contained a smaller amount of water (just that carried over from the first stage desalter).

The radar plots shown in Fig. 5b were obtained upstream of a wash water inlet (at location 4 as shown in Fig. 4, denoted in Fig. 5b by 'U') and downstream of a wash water inlet but upstream of a mixing valve (at location 5 as shown in Fig. 4, denoted in Fig. 5b by 'M'). It can be seen from the plot that there are two distinct phases of water and oil.

The radar plots shown in Fig. 5c were obtained upstream of a wash water inlet (at location 4 as shown in Fig. 4, denoted in Fig. 5c by 'U') and downstream of a mixing valve (downstream of mixing valve 120b as shown in Fig. 4, denoted in Fig. 5c by 'D'). There is minimal difference between the backscatter counts without wash water and with wash water in the crude oil. This indicates that optimum mixing is already taking place in the line in the second stage.

Since the results indicate that good mixing occurs between the crude oil and wash water, then there is no need to improve the mixing. Optimization studies may nonetheless be carried out with a view to improving the efficiency of the desalting process.

The results demonstrate that the use of neutron backscattering is an effective tool for optimizing a desalting process.

## Claims

1. A method for optimizing a desalting process in which a hydrocarbon feedstock is passed to a desalter through a line under a set of conditions, the hydrocarbon feedstock containing a hydrocarbon fluid, water and a salt, said method comprising:
obtaining spectra of the hydrocarbon feedstock under a plurality of environments in the line;
comparing the spectra; and
based on the comparison of the spectra, either modifying or maintaining the set of conditions under which the hydrocarbon feedstock is passed to the desalter;
wherein the spectra are obtained using neutron backscattering.

2. The method of Claim 1, wherein the hydrocarbon feedstock comprises crude oil and/or sodium chloride.

3. The method of Claim 1 or Claim 2, wherein spectra are obtained at a plurality of locations on the line.

4. The method of Claim 3, wherein spectra are obtained at longitudinally spaced locations along the line.

5. The method of Claim 4, wherein the line comprises a wash water inlet and spectra are obtained at a location upstream of the wash water inlet and at a location downstream of the wash water inlet.

6. The method of Claim 4 or Claim 5, wherein the line comprises a mixing valve and spectra are obtained at a location upstream of the mixing valve and at a location downstream of the mixing valve.

7. The method of any of Claims 1 to 6, wherein spectra, preferably at least four spectra, are obtained at radially spaced locations around the line.

8. The method of Claim 7, wherein a plurality of sets of spectra are obtained at longitudinally spaced locations on the line, each set of spectra obtained at radially spaced locations on the line.

9. The method of any of Claims 1-8, wherein spectra are obtained at different points in time from the same location on the line, preferably wherein spectra are obtained at a point in time before the addition of wash water to the line and at a point in time after the addition of wash water to the line.

10. The method of any of Claims 1-9, wherein, based on the comparison of the spectra, the set of conditions under which the hydrocarbon feedstock is passed to the desalter are modified.

11. The method of Claim 10, wherein the method further comprises testing the effect of the modified conditions, wherein testing preferably comprises obtaining further spectra of the hydrocarbon feedstock to determine whether the modified conditions have an effect on mixing of the hydrocarbon fluid with water and, if the modified conditions do have an effect on mixing of the hydrocarbon fluid with water, maintaining, reversing or further modifying the set of conditions under which the hydrocarbon feedstock is passed to the desalter; and wherein the steps of obtaining spectra, comparing spectra and modifying conditions are preferably repeated at least three times.

12. The method of any of Claims 1-11, wherein the set of conditions under which the hydrocarbon feedstock is passed to the desalter are modified by making changes to the wash water inlet, by making changes to the mixing valve, by adjusting the introduction of additive components into the line, by adjusting the temperature and pressure in the line, or by making changes to the hydrocarbon feedstock.

13. The method of any of Claims 1-12, wherein multiple desalting stages are present in the desalting process, and the steps of obtaining spectra, comparing spectra and optionally modifying conditions are carried out on each of the desalting stages.

14. An apparatus comprising:
a desalter;
a line through which a hydrocarbon feedstock is passed to the desalter, the hydrocarbon feedstock containing a hydrocarbon fluid, water and a salt; and
a neutron backscatter spectrometer positioned so as to obtain a spectrum of the hydrocarbon feedstock in the line.

15. Use of neutron backscattering performed on a line through which a hydrocarbon feedstock is passed to a desalter for optimizing the desalting of a hydrocarbon feedstock in a desalting process, the hydrocarbon feedstock containing a hydrocarbon fluid, water and a salt.

## Patentansprüche

1. Verfahren zum Optimieren eines Entsalzungsprozesses, bei dem ein Kohlenwasserstoffeinsatzmaterial durch eine Leitung unter einem Satz von Bedingungen zu einem Entsalzer geleitet wird, wobei das Kohlenwasserstoffeinsatzmaterial ein Kohlenwasserstofffluid, Wasser und ein Salz enthält, wobei das genannte Verfahren Folgendes beinhaltet:
Gewinnen von Spektren des Kohlenwasserstoffeinsatzmaterials unter mehreren Umgebungen in der Leitung;
Vergleichen der Spektren; und
Modifizieren oder Halten, auf der Basis des Vergleichs der Spektren, des Satzes von Bedingungen, unter denen das Kohlenwasserstoffeinsatzmaterial zum Entsalzer geleitet wird;
wobei die Spektren mit Hilfe von Neutronenrückstreuung gewonnen werden.

2. Verfahren nach Anspruch 1, wobei das Kohlenwasserstoffeinsatzmaterial Rohöl und/oder Natriumchlorid umfasst.

3. Verfahren nach Anspruch 1 oder Anspruch 2, wobei Spektren an mehreren Stellen auf der Leitung gewonnen werden.

4. Verfahren nach Anspruch 3, wobei die Spektren an longitudinal beabstandeten Stellen entlang der Leitung gewonnen werden.

5. Verfahren nach Anspruch 4, wobei die Leitung einen Waschwassereinlass umfasst und Spektren an einer Stelle stromaufwärts des Waschwassereinlasses und an einer Stelle stromabwärts des Waschwassereinlasses gewonnen werden.

6. Verfahren nach Anspruch 4 oder Anspruch 5, wobei die Leitung ein Mischventil umfasst und Spektren an einer Stelle stromaufwärts des Mischventils und an einer Stelle stromabwärts des Mischventils gewonnen werden.

7. Verfahren nach einem der Ansprüche 1 bis 6, wobei Spektren, vorzugsweise wenigstens vier Spektren, an radial beabstandeten Stellen um die Leitung gewonnen werden.

8. Verfahren nach Anspruch 7, wobei mehrere Sätze von Spektren an longitudinal beabstandeten Stellen auf der Leitung gewonnen werden, wobei jeder Satz von Spektren an radial beabstandeten Stellen auf der Leitung gewonnen wird.

9. Verfahren nach einem der Ansprüche 1-8, wobei Spektren zu unterschiedlichen Zeitpunkten von derselben Stelle auf der Leitung gewonnen werden, wobei vorzugsweise Spektren zu einem Zeitpunkt vor der Zugabe von Waschwasser zu der Leitung und zu einem Zeitpunkt nach der Zugabe von Waschwasser zu der Leitung gewonnen werden.

10. Verfahren nach einem der Ansprüche 1-9, wobei auf der Basis des Vergleichs der Spektren der Satz von Bedingungen modifiziert wird, unter denen das Kohlenwasserstoffeinsatzmaterial zum Entsalzer geleitet wird.

11. Verfahren nach Anspruch 10, wobei das Verfahren ferner das Testen der Auswirkung der modifizierten Bedingungen beinhaltet, wobei das Testen vorzugsweise das Gewinnen weiterer Spektren des Kohlenwasserstoffeinsatzmaterials beinhaltet, um festzustellen, ob die modifizierten Bedingungen eine Auswirkung auf das Mischen des Kohlenwasserstofffluids mit Wasser haben und, wenn die modifizierten Bedingungen eine Auswirkung auf das Mischen des Kohlenwasserstofffluids mit Wasser haben, Halten, Umkehren oder weiter Modifizieren des Satzes von Bedingungen, unter denen das Kohlenwasserstoffeinsatzmaterial zum Entsalzer geleitet wird; und
wobei die Schritte des Gewinnens von Spektren, des Vergleichens von Spektren und des Modifizierens von Bedingungen vorzugsweise wenigstens dreimal wiederholt werden.

12. Verfahren nach einem der Ansprüche 1-11, wobei der Satz von Bedingungen, unter denen das Kohlenwasserstoffeinsatzmaterial zum Entsalzer geleitet wird, durch Vornehmen von Änderungen am Waschwassereinlass, durch Vornehmen von Änderungen am Mischventil, durch Justieren des Einleitens von Additivkomponenten in die Leitung, durch Justieren der Temperatur und des Drucks in der Leitung oder durch Vornehmen von Änderungen am Kohlenwasserstoffeinsatzmaterial modifiziert werden.

13. Verfahren nach einem der Ansprüche 1-12, wobei der Entsalzungsprozess mehrere Entsalzungsstufen beinhaltet und die Schritte des Gewinnens von Spektren, des Vergleichens von Spektren und des optionalen Modifizierens von Bedingungen in jeder der Entsalzungsstufen durchgeführt werden.

14. Vorrichtung, die Folgendes umfasst:
einen Entsalzer;
eine Leitung, durch die ein Kohlenwasserstoffeinsatzmaterial zum Entsalzer geleitet wird, wobei das Kohlenwasserstoffeinsatzmaterial ein Kohlenwasserstofffluid, Wasser und ein Salz enthält; und
ein Neutronenrückstreuspektrometer, das so positioniert ist, dass es ein Spektrum des Kohlenwasserstoffeinsatzmaterials in der Leitung gewinnt.

15. Verwendung von Neutronenrückstreuung, durchgeführt auf einer Leitung, durch die ein Kohlenwasserstoffeinsatzmaterial zu einem Entsalzer geleitet wird, zum Optimieren der Entsalzung eines Kohlenwasserstoffeinsatzmaterials in einem Entsalzungsprozess, wobei das Kohlenwasserstoffeinsatzmaterial ein Kohlenwasserstofffluid, Wasser und ein Salz enthält.

## Revendications

1. Méthode d'optimisation d'un procédé de dessalage dans lequel une charge d'alimentation en hydrocarbure est passée vers un dessaleur à travers une ligne dans un ensemble de conditions, la charge d'alimentation en hydrocarbure contenant un fluide d'hydrocarbure, de l'eau et un sel, ladite méthode comprenant :
l'obtention de spectres de la charge d'alimentation en hydrocarbure dans une pluralité d'environnements dans la ligne ;
la comparaison des spectres ; et
sur la base de la comparaison des spectres, soit la modification ou le maintien de l'ensemble des conditions dans lesquelles la charge d'alimentation en hydrocarbure est passée vers le dessaleur ;
dans laquelle les spectres sont obtenus en utilisant la rétrodiffusion des neutrons.

2. Méthode selon la revendication 1, dans laquelle la charge d'alimentation en hydrocarbure comprend une huile brute et/ou du chlorure de sodium.

3. Méthode selon la revendication 1 ou la revendication 2, dans laquelle les spectres sont obtenus au niveau d'une pluralité d'emplacements sur la ligne.

4. Méthode selon la revendication 3, dans laquelle les spectres sont obtenus au niveau d'emplacements espacés de manière longitudinale le long de la ligne.

5. Méthode selon la revendication 4, dans laquelle la ligne comprend une entrée d'eau de lavage et des spectres sont obtenus au niveau d'un emplacement en amont de l'entrée d'eau de lavage et au niveau d'un emplacement en aval de l'entrée d'eau de lavage.

6. Méthode selon la revendication 4 ou la revendication 5, dans laquelle la ligne comprend une vanne mélangeuse et des spectres sont obtenus au niveau d'un emplacement en amont de la vanne mélangeuse et au niveau d'un emplacement en aval de la vanne mélangeuse.

7. Méthode selon l'une quelconque des revendications 1 à 6, dans laquelle des spectres, préférablement au moins quatre spectres, sont obtenus au niveau d'emplacements espacés de manière radiale autour de la ligne.

8. Méthode selon la revendication 7, dans laquelle une pluralité d'ensembles de spectres sont obtenus au niveau d'emplacements espacés de manière longitudinale sur la ligne, chaque ensemble de spectres obtenu au niveau d'emplacements espacés de manière radiale sur la ligne.

9. Méthode selon l'une quelconque des revendications 1 à 8, dans laquelle des spectres sont obtenus à différents points dans le temps à partir du même emplacement sur la ligne, préférablement dans laquelle des spectres sont obtenus à un point dans le temps avant l'ajout d'eau de lavage vers la ligne et à un point dans le temps après l'ajout d'eau de lavage vers la ligne.

10. Méthode selon l'une quelconque des revendications 1 à 9, dans laquelle, sur la base de la comparaison des spectres, l'ensemble de conditions dans lesquelles la charge d'alimentation en hydrocarbure est passée vers le dessaleur est modifié.

11. Méthode selon la revendication 10, dans laquelle la méthode comprend en outre le fait de tester l'effet des conditions modifiées, dans laquelle le fait de tester comprend préférablement l'obtention de spectres supplémentaires de la charge d'alimentation en hydrocarbure pour déterminer si les conditions modifiées ont un effet sur le mélange du fluide d'hydrocarbure avec de l'eau et, si les conditions modifiées ont un effet sur le mélange du fluide d'hydrocarbure avec de l'eau, le maintien, le renversement ou la modification supplémentaire de l'ensemble de conditions dans lesquelles la charge d'alimentation en hydrocarbure est passée vers le dessaleur ; et
dans laquelle les étapes d'obtention de spectres, de comparaison de spectres et de modification de conditions sont préférablement répétées au moins trois fois.

12. Méthode selon l'une quelconque des revendications 1 à 11, dans laquelle l'ensemble de conditions dans lesquelles la charge d'alimentation en hydrocarbure est passée vers le dessaleur est modifié en apportant des modifications à l'entrée d'eau de lavage, en apportant des modifications à la vanne mélangeuse, en ajustant l'introduction de composants additifs dans la ligne, en ajustant la température et la pression dans la ligne, ou en apportant des modifications à la charge d'alimentation en hydrocarbure.

13. Méthode selon l'une quelconque des revendications 1 à 12, dans laquelle des étapes de dessalage multiples sont présentes dans le procédé de dessalage, et les étapes d'obtention de spectres, de comparaison de spectres et facultativement de modification de conditions sont réalisées lors de chacune des étapes de dessalage.

14. Appareil comprenant :
un dessaleur ;
une ligne à travers laquelle une charge d'alimentation en hydrocarbure est passée vers le dessaleur, la charge d'alimentation en hydrocarbure contenant un fluide d'hydrocarbure, de l'eau et un sel ; et
un spectromètre à rétrodiffusion des neutrons positionné de manière à obtenir un spectre de la charge d'alimentation en hydrocarbure dans la ligne.

15. Utilisation de la rétrodiffusion des neutrons réalisée sur une ligne à travers laquelle une ligne d'alimentation en hydrocarbure est passée vers un dessaleur pour optimiser le dessalage d'une charge d'alimentation en hydrocarbure dans un procédé de dessalage, la charge d'alimentation en hydrocarbure contenant un fluide d'hydrocarbure, de l'eau et un sel.
